# EUROPEAN PATENT SPECIFICATION

(11) **EP 1 898 698 B1**
(45) Date of publication and mention of the grant of the patent: **21.05.2014**
(21) Application number: 06742494.5
(22) Date of filing: 29.06.2006
(51) Int. Cl.: G01N 33/50

(54) **NON-HUMAN MAMMALIAN ARTHRITIS MODEL FEATURING HUMAN ANTIBODIES AGAINST CITRULLINATED PROTEINS**
NICHT-HUMANES SÄUGETIER-MODELL FÜR ARTHRITIS, DAS HUMANE ANTIKÖRPER GEGEN CITRULLINIERTE PROTEINE AUFWEIST
MODELE ARTHRITIQUE MAMMIFERE NON HUMAIN COMPORTANT DES ANTICORPS HUMAINES CONTER DES PROTEINES CONTENANT DE LA CITRULLINE

(30) Priority: 29.06.2005 US 695833 P; 01.07.2005 US 696361 P
(43) Date of publication of application: 19.03.2008
(73) Proprietor: Genmab A/S, 1260 Copenhagen K (DK)
(72) Inventor: TOES, Rene, 2313 DN Leiden (NL); HUIZINGA, Thomas, 2318 ND Leiden (NL); MOLENAAR, Miranda, 3601 CL Maarssen (NL); PARREN, Paul, 3984 PR Odijk (NL); VAN DE WINKEL, Jan, 3707 CK Zeist (NL)
(74) Representative: Høiberg A/S
(86) International application number: PCT/DK2006/000384
(87) International publication number: WO 2007/000169

(56) References cited:
- WO-A2-2004/078098
- IWAKI-EGAWA S ET AL: "Production of anti-CCP antibodies and matrix metalloproteinase-3 by human rheumatoid arthritis synovial tissues using SCID mice" ANNALS OF THE RHEUMATIC DISEASES 2005 UNITED KINGDOM, vol. 64, no. 7, 15 June 2005 (2005-06-15), pages 1094-1095, XP002408812 ISSN: 0003-4967
- MATSUNO H ET AL: "TREATMENT OF RHEUMATOID SYNOVITIS WITH ANTI-RESHAPING HUMAN INTERLEUKIN-6 RECEPTOR MONOCLONAL ANTIBODY USE OF RHEUMATOID ARTHRITIS TISSUE IMPLANTS IN THE SCID MOUSE MODEL" ARTHRITIS AND RHEUMATISM, LIPPINCOTT, PHILADELPHIA, US, vol. 41, no. 11, November 1998 (1998-11), pages 2014-2021, XP001069959 ISSN: 0004-3591
- SAKAI K ET AL: "POTENTIAL WITHDRAWAL OF RHEUMATOID SYNOVIUM BY THE INDUCTION OF APOPTOSIS USING A NOVEL IN VIVO MODEL OF RHEUMATOID ARTHRITIS" ARTHRITIS AND RHEUMATISM, LIPPINCOTT, PHILADELPHIA, US, vol. 41, no. 7, July 1998 (1998-07), pages 1251-1257, XP002927780 ISSN: 0004-3591
- DATABASE BIOSIS [Online] BIOSCIENCES INFORMATION SERVICE, PHILADELPHIA, PA, US; March 2005 (2005-03), KUHN KRISTINE A ET AL: "Antibodies to citrulline-modified proteins enhance tissue injury in inflammatory arthritis" XP002408887 Database accession no. PREV200510325980 & FASEB JOURNAL, vol. 19, no. 4, Suppl. S, Part 1, March 2005 (2005-03), page A913, EXPERIMENTAL BIOLOGY 2005 MEETING/35TH INTERNATIONAL CONGRESS OF PHYSIOLOGICAL SCIENCES; SAN DIEGO, CA, USA; MARCH 31 -APRIL 06, 2005 ISSN: 0892-6638
- VOSSENAAR ERIK R ET AL: "Absence of citrulline-specific autoantibodies in animal models of autoimmunity." ARTHRITIS AND RHEUMATISM. JUL 2004, vol. 50, no. 7, July 2004 (2004-07), pages 2370-2372, XP002408813 ISSN: 0004-3591
- VAN GAALEN F ET AL: "The devil in the details: The emerging role of anticitrulline autoimmunity in rheumatoid arthritis" JOURNAL OF IMMUNOLOGY 01 NOV 2005 UNITED STATES, vol. 175, no. 9, 1 November 2005 (2005-11-01), pages 5575-5580, XP002408824 ISSN: 0022-1767
- PATEL DHAVALKUMAR D: "B cell-ablative therapy for the treatment of autoimmune diseases", ARTHRITIS & RHEUMATISM, JOHN WILEY & SONS, INC, US, vol. 46, no. 8, 1 August 2002 (2002-08-01) , pages 1984-1985, XP002456841, ISSN: 0004-3591, DOI: 10.1002/ART.10476
- R. J. LOONEY: "B cells as a therapeutic target in autoimmune diseases other than rheumatoid arthritis", RHEUMATOLOGY, vol. 44, no. SUPPL_2, 1 May 2005 (2005-05-01), pages II13-II17, XP055062468, ISSN: 1462-0324, DOI: 10.1093/rheumatology/keh618
- J. C. W. EDWARDS ET AL: "Prospects for B-cell-targeted therapy in autoimmune disease", RHEUMATOLOGY, vol. 44, no. 2, 1 February 2005 (2005-02-01), pages 151-156, XP055062540, ISSN: 1462-0324, DOI: 10.1093/rheumatology/keh446

## Description

### FIELD OF THE INVENTION

The present invention relates to the use of a non-human mammalian disease model, wherein the non-human mammal has been implanted with human synovial tissue or other human inflamed tissue containing anti-CCP (cyclic citrullinated peptide) antibody producing cells for (i) testing the efficacy of a therapeutic agent for the prevention or treatment of a disease associated with anti-CCP antibodies, and (ii) identifying a therapeutic agent useful for the prevention or treatment of a disease associated with anti-CCP antibodies. In a particular embodiment the non-human mammalian disease model is a mouse, such as a SCID mouse, and the disease associated with anti-CCP antibodies is RA (rheumatoid arthritis).

### BACKGROUND OF THE INVENTION

Anti-CCP antibodies play a role in arthritis and are currently widely recognized as the most disease-specific marker in RA (Schellekens GA, et al., (2000) "The diagnostic properties of rheumatoid arthritis antibodies recognizing a cyclic citrullinated peptide" Arthritis Rheum 43: 155-163, and Vossenaar ER, et al. (2004) "Anti-CCP antibodies, a highly specific marker for (early) rheumatoid arthritis (RA)" Clin Appl Immunol Rev 4: 239-262) and have also been shown to have important prognostic value (van Gaalen FA, et al. (2004) "Autoantibodies to cyclic citrullinated peptides predict progression to rheumatoid arthritis in patients with undifferentiated arthritis: a prospective cohort study" Arthritis Rheum 50: 709-715).

Remarkably, within the group of anti-CCP-positive patients with undifferentiated arthritis, 95-98% will develop RA (positive predictive value of 93%, odds ratio of 37.8). Importantly, multivariate analyses, in which the anti-CCP antibodies were added to the 7 ACR (American College of Rheumatology) criteria, showed that the presence of anti-CCP antibodies were the most important predictive factor with an odds ratio of 38.6 (95% CI (confidence interval) 9.9 - 151.0). Moreover, anti-CCP antibodies can be detected in RA patients several years before clinical symptoms occur, indicating that the induction of anti-CCP antibodies precede the development of RA (Nielen NN, et al. (2004) "Specific autoantibodies precede the symptoms of rheumatoid arthritis: a study of serial measurements in blood donors" Arthritis Rheum 50: 380-386). All these findings suggest that anti-CCP antibodies play a role in the induction of arthritis and have an influence on the severity of arthritis.

Furthermore, a number of observations suggest that anti-CCP antibodies play a role in the pathophysiology of RA. Firstly, high anti-CCP antibody titers are associated with severe and erosive disease progression (Vencovsky J, et al. (2003) "Autoantibodies can be prognostic markers of an erosive disease in early rheumatoid arthritis" Ann Rheum Dis 62: 427-430). Secondly, decrease in RF (rheumatoid factor) and anti-CCP titers after B cell depletion by anti-CD20 therapy suggests an important function for B cells in the pathophysiology of RA (Cambridge G, et al. (2003) "Serologic changes following B cell lymphocyte depletion therapy for rheumatoid arthritis" Arthritis Rheum 48: 2146-2154). A decrease in anti-CCP titers was also observed in RA patients treated with anti-TNFα therapy in combination with low-dose methothrexate (Alessandri C, et al. (2004) "Decrease of anti-cyclic citrullinated peptide antibodies and rheumatoid factor following anti-TNFalpha therapy (infliximab) in rheumatoid arthritis is associated with clinical improvement" Ann Rheum Dis 63:1218-1221). In this study, changes in anti-CCP titers and clinical responses were correlated; patients with best clinical improvement during the therapy had the lowest anti-CCP titers at baseline and showed strongest decrease in titer upon therapy.

Anti-CCP antibodies recognize proteins containing the nonstandard amino acid citrulline, which is the product of posttranslational modification of arginine residues by the calcium-dependent peptidyl arginine deiminase (PAD) enzymes (Vossenaar ER, et al. (2003) "PAD, a growing family of citrullinated enzymes: genes, features and involvement in disease" Bioassays 25: 1106-1118). An example of a citrullinated protein present in inflamed RA joints is extravascular fibrin (Masson-Bessière C, et al. (2001) "The major synovial targets of the rheumatoid arthritis-specific antifilaggrin autoantibodies are deiminated forms of the α- and β-chains of fibrin" J Immunol 166: 4177-4184). The inflamed synovium also contains many PAD2-expressing macrophages and sometimes PAD4-expressing granulocytes (Vossenaar ER, et al. (2004) "Expression and activity of citrullinating PAD enzymes in monocytes and macrophages" Ann Rheum Dis 63: 373-381). Normally, PAD enzymes are present intracellularly in their inactive form, but when the cells are dying, for example as a result of extensive oxidative stress in the inflamed synovium, PAD enzymes might leak out from the cells, become activated upon changes in calcium concentration and deiminate extracellular proteins (Vossenaar ER, *et al.* (2003) supra).

Several animal models have been used to study mechanisms of inflammation in RA (Morgan DW, et al. (1999) "In vivo models of inflammation" Birkhauser Verlag, Basel, Switzerland), but none of them have been successfully applied to study the role of anti-CCP antibodies in the pathophysiology of this disease. Recently, it has been shown that synovial proteins, like fibrin, are citrullinated during inflammation in both an acute (streptococcal cell wall arthritis) and a chronic (collagen-induced arthritis) mouse model for arthritis (Vossenaar ER, et al. (2003) "Citrullination of synovial proteins in murine models of rheumatoid arthritis" Arthritis Rheum 48: 2489-2500). However, in contrast to the human disease, the mice did not develop antibodies against citrullinated proteins.

Tanaka K et al. (2003) "Inhibitory effects of an anti-rheumatic agent T-614 on immunoglobulin production by cultured B cells and rheumatoid synovial tissue or other human inflamed tissues engrafted into SCID mice" Rheumatology 42:1365-1371 disclose SCID mice engrafted with human RA tissue (SCID-HuRag mice) and show that treatment of the mice with T-614 (a cytokine production inhibitor) decreases the high levels of human IgG observed in SCID-HuRag mice.

Iwaki-Egawa S et al. (2005) "Production of anti-CCP antibodies and matrix metalloproteinase-3 by human rheumatoid arthritis synovial tissues using SCID mice" Ann Rheum Dis 64(7): 1094-1095 disclose that SCID-HuRAg mice produce anti-CCP antibodies at a certain point of time and suggest to use said mice in the study and development of new drugs for rheumatoid arthritis.

Matsuno H et al. (1998) "Treatment of rheumatoid synovitis with anti-reshaping human interleukin-6 receptor monoclonal antibody" Arthritis Rheum 41(11): 2014-2021 as well as Sakai K et al. (1998) "Potential withdrawal of rheumatoid synovium by the induction of apoptosis using a novel in vivo model of rheumatoid arthritis" Arthritis Rheum 41 (7): 1251-1257 disclose the use of SCID-HuRAg mice for drug screening.

Patel D D (2002) "B cell-ablative therapy for the treatment of autoimmune diseases" Arthritis Rheum 46(8): 1984-1985; Looney R J (2005) "B cells as a therapeutic target in autoimmune diseases other than rheumatoid arthritis" Rheumatol 44(Suppl. 2): ii13-ii17; and Edwards J & Cambridge G (2005) "Prospects for B-cell-targeted therapy in autoimmune disease" Rheumatol 44(2): 151-156 disclose the use of anti-CD20 antibodies such as rituximab for the treatment of rheumatoid arthritis.

While a variety of transgenic and knock-out mice have been found to develop autoimmunity, no animal model producing anti-CCP antibodies or developing spontaneous inflammatory arthritis has been disclosed.

Accordingly, a continued need exists in the art for additional animal models, compositions and methods to enable the analysis of cellular processes in a disease associated with anti-CCP antibodies, the study of the role of anti-CCP antibodies in the induction and progression of a disease associated with anti-CCP antibodies, and the test and identification of therapeutic agents for the prevention or treatment of a disease associated with anti-CCP antibodies.

### SUMMARY OF THE INVENTION

The present invention provides a non-human mammalian disease model as a unique tool to test the effectiveness of novel anti-inflammatory therapies with anti-CD20 antibodies.

The term "non-human mammalian disease model" as used herein means all mammals, except humans.

The specification relates to the use of a non-human mammalian disease model, wherein the non-human mammal has been implanted with human synovial tissue or other human inflamed tissue containing anti-CCP antibody producing cells, for analyzing cellular processes in a disease associated with anti-CCP antibodies.

Furthermore, the specification relates to the use of a non-human mammalian disease model, wherein the non-human mammal has been implanted with human synovial tissue or other human inflamed tissue containing anti-CCP antibody producing cells, for studying the role of anti-CCP antibodies in the induction and progression of a disease associated with anti-CCP antibodies.

In one aspect the invention relates to the use of a non-human mammalian disease model, wherein the non-human mammal has been implanted with human synovial tissue or other human inflamed tissue containing anti-CCP antibody producing cells, for testing the efficacy of a therapeutic agent for the prevention or treatment of a disease associated with anti-CCP antibodies.

In a further aspect the invention relates to the use of a non-human mammalian disease model, wherein the non-human mammal has been implanted with human synovial tissue or other human inflamed tissue containing anti-CCP antibody producing cells, for identifying a therapeutic agent useful for the prevention or treatment of a disease associated with anti-CCP antibodies.

The efficacy of the therapeutic agent may be measured or the therapeutic agent may be identified by its ability to inhibit a marker of a disease associated with anti-CCP antibodies.

According to the invention the efficacy of the therapeutic agent is measured or the therapeutic agent is identified by its ability to reduce the concentration or activity of anti-CCP antibodies in the serum of the non-human mammalian model.

The efficacy of the therapeutic agent may be measured or the therapeutic agent may be identified by its ability to reduce the concentration or activity of anti-CCP antibodies by binding thereto in the serum of the non-human mammalian model.

The efficacy of the therapeutic agent may be measured or the therapeutic agent may be identified by its ability to inhibit production of anti-CCP antibodies or to knock-out anti-CCP producing cells in the non-human mammalian model.

The efficacy of the therapeutic agent may be measured or the therapeutic agent may be identified by its ability to reduce the Tender Joint Count (TJC) and/or Swollen Joint Count (SJC) of the non-human mammalian model. TJC and SJC have been developed by the American College of Rheumatology (ACR) as disease activity measures in RA.

The efficacy of the therapeutic agent may be measured or the therapeutic agent may be identified by its ability to reduce or inhibit one or more of the disease markers or conditions selected from the group consisting of:
(i) rheumatoid factor (RF), such as IgM-RF or IgG-RF,
(ii) acute phase proteins, such as serum amyloid protein (SAP),
(iii) ankylosis,
(iv) cartilage degradation,
(v) bone erosions, and
(vi) cellular infiltrate.

The disease associated with anti-CCP antibodies may be arthritis, such as a disease selected from the group consisting of psoriatic arthritis, MCTD (mixed connective tissue disease), crystal induced arthritis, reactive arthritis, spondylarthropathy, osteoarthritis, sarcoidosis, palindromic rheumatism, post traumatic arthritis, malignancy related arthritis, septic arthritis, lyme arthritis, SLE (systemic lupus erythematosus), juvenile chronic arthritis, other forms of juvenile arthritis, undifferentiated arthritis, and arthritis e *causa incognita.*

According to the invention the disease associated with anti-CCP antibodies is RA (rheumatoid arthritis).

The disease associated with anti-CCP antibodies may be vasculitis.

The disease associated with anti-CCP antibodies may be selected from the group consisting of primary vasculitides, e.g. small vessel vasculitis, such as Wegener's granulomatosis, or secondary vasculitides, e.g. rheumatoid vasculitis.

The therapeutic agent to be tested or identified may be an anti-arthritis agent or an anti-inflammatory agent.

The therapeutic agent to be tested or identified may be an antibody or an antibody fragment. The antibody can be polyclonal or monoclonal. The antibody can be chimeric, humanized or fully human. Furthermore, it could be a bi-specific antibody or a combination of two or more antibodies. Other examples of therapeutic agents are small molecules, peptides, nucleic acids, other binding molecules, etc.

The antibody may be selected from the group consisting of anti-IL-15 antibodies, anti-TNF-alpha antibodies, anti-IL-1 antibodies, anti-IL-1R antibodies, anti-IL-2Ralpha antibodies, and antibodies against IL-1 accessory protein. Further non-limiting examples are anti-IL-6Ralpha antibodies, anti-gp130 antibodies, and anti-CD38 antibodies.

According to the invention the antibody is an anti-CD20 antibody, such as one of the antibodies disclosed in WO 2004/035607 (2F2, 7D8 and 11B8), rituximab, tositumomab, 2H7.v16 as disclosed in WO 2004/56312, IMMU-106 as disclosed in WO 2003/68821, or TRU-015 as disclosed in US 2003/0118592.

The antibody may be an anti-IL2Ralpha antibody, such as daclizumab, basiliximab, or one of the antibodies disclosed in WO 2004/045512 (AB1, AB7, AB11, or AB12), or an anti-IL15 antibody, such as 146B7 as disclosed in WO 03/017935.

In one embodiment of the invention the non-human mammal is a mouse, such as a SCID mouse or a nude mouse.

The term "SCID mouse" as used herein means a mutant mouse strain producing very few mature B and T cells due to a failure of DNA rearrangement in the developing lymphocytes of the mice. SCID is an abbreviation for severe combined immune deficient.

The non-human mammal may have been implanted with human synovial tissue or other human inflamed tissue containing anti-CCP antibody producing cells derived from a patient suffering from arthritis, such as from a patient suffering from a disease selected from the group consisting of psoriatic arthritis, MCTD, crystal induced arthritis, reactive arthritis, spondylarthropathy, osteoarthritis, sarcoidosis, palindromic rheumatism, post traumatic arthritis, malignancy related arthritis, septic arthritis, lyme arthritis, SLE, juvenile chronic arthritis, other forms of juvenile arthritis, undifferentiated arthritis, and arthritis e *causa incognita.*

In one embodiment of the invention the non-human mammal has been implanted with human synovial tissue or other human inflamed tissue containing anti-CCP antibody producing cells derived from a patient suffering from RA.

In one embodiment of the invention an agent for stimulating anti-CCP antibody production, such as LPS (lipopolysaccharide) or T cell-derived cytokines, such as IL-4, IFN-Y (interferon-gamma) or IL-10, has been added to the human synovial tissue or other human inflamed tissue containing anti-CCP antibody producing cells prior to implantation thereof.

In one embodiment of the invention the non-human mammal has been administered with an agent for stimulating anti-CCP antibody production, such as LPS (lipopolysaccharide) or T cell-derived cytokines, such as IL-4, IFN-Y (interferon-gamma) or IL-10, prior to, during or after implantation of the human synovial tissue or other human inflamed tissue containing anti-CCP antibody producing cells.

In one embodiment of the invention the non-human mammal has been implanted with human synovial tissue or other human inflamed tissue containing anti-CCP antibody producing cells, wherein arthritis is induced by intra-articular injection of an inflammation inducing agent.

In one embodiment of the invention the inflammation inducing agent is selected from the group consisting of LPS, streptococcal wall-debris, immune complexes, cytokine-producing recombinant viruses, including recombinant adenovirus, recombinant adeno-associated virus, and recombinant retroviruses.

In one embodiment of the invention the non-human mammal has been implanted with human synovial tissue or other human inflamed tissue containing anti-CCP antibody producing cells, wherein citrullinated proteins are generated in the articular joint by intra-articular injection of recombinant viruses encoding citrunillating enzymes of human, vertebrate or non-vertebrate origin, including peptidyl arginine deiminase 1-6, by injection of other vectors encoding such enzymes, or by injection of protein representing such enzymes.

Other aspects and advantages of the present invention are described further in the following detailed description.

### BRIEF DESCRIPTION OF THE DRAWINGS

*Figure 1* shows immunohistochemical staining for human van Willebrand factor and murine CD31 on acetone-fixed cryosections of non-implanted synovial tissue and a synovial graft explanted from SCID-mouse 7, 17, and 27 days after implantation. Sections were counterstained with hematoxylin. Magnification: 125 x.
*Figure* 2 shows immunohistochemical staining for human cell-specific markers on acetone-fixed cryosections of non-implanted synovial tissue and a synovial graft explanted from SCID-mouse 27 days after implantation. Sections were counterstained with hematoxylin. Magnification: 125 x.
*Figure 3* shows immunohistochemical staining for proliferation marker Ki-67 on acetone-fixed cryosections of non-implanted synovial tissue and a synovial graft explanted from SCID-mouse 7, 17, and 27 days after implantation. Sections were counterstained with hematoxylin. Magnification: 125 x.
*Figure 4* shows the presence of anti-CCP antibodies in the sera of SCID-mice implanted with anti-CCP+ or anti-CCP- human synovial tissue as measured by ELISA. White bars: plate coated with cyclic peptide containing arginine. Black bars: plate coated with CCP. After implantation: pooled sera 1 week and 2 weeks after implantation. Positive control: serum of anti-CCP+ patient.
*Figure* 5 shows a simulation curve for IgG production by synovial xenograft in an RA SCID mouse model. The anti-CCP antibodies form part of the IgG and will follow the same kinetics as shown for IgG.

### DETAILED DESCRIPTION OF THE INVENTION

It is believed that anti-CCP antibodies are involved in the induction or progression of arthritis. A possible mechanism by which anti-CCP antibodies could contribute to disease progression is as follows. An initial small inflammation might cause death of PAD-expressing cells, release of PAD enzymes and the generation of citrullinated proteins in the synovium. In anti-CCP positive patients, the locally produced anti-CCP antibodies would bind citrullinated proteins and form immune complexes, which would subsequently cause activation of inflammatory cells and production of pro-inflammatory cytokines. Next, the cytokines would promote influx of more inflammatory cells into the synovium which would eventually die and cause the production of more citrullinated proteins. In this way, anti-CCP antibodies might contribute to the perpetuation of the inflammation and the chronic nature of the disease.

The present specification provides a useful tool for investigating in vivo properties of novel therapies, such as treatment with monoclonal antibodies directed against a particular cell type, cytokine, etc. Monitoring of changes in the composition and magnitude of cellular infiltrate in the graft upon treatment with novel therapies can also provide information on interactions between cells and immune mediators. The presence of anti-CCP antibodies in sera of the non-human mammals implanted with human synovial tissue allows investigations on the role of these antibodies in the induction or progression of RA and other diseases in which anti-CCP antibodies play a role.

Various aspects of the invention are described in further detail in the following examples.

### EXAMPLES

### Example 1 - Implantation of synovial tissue in mice

SCID-mice, strain C.B.-17/IcrCrl-SCID-bg, male / female, 4-12 weeks, purchased from Charles River Nederland (Maastricht, the Netherlands) were kept in IVC cages under standard conditions of temperature and light, and were fed laboratory chow and water ad libitum. The study was approved by the Ethics Committee of the Leiden University Medical Center-Academic Hospital Leiden (ADEC), the Netherlands.

Before implantation the mice were anesthetized by intraperitoneal injection of ketamine (NIMATEK, EuroVet) and xylazine (Rompun, Bayer) in a v/v ratio of 1:1. A small incision of the skin was made using surgical scissors. Inflamed synovial tissue of a patient with RA undergoing joint replacement surgery was implanted subcutaneously as a cluster of six small fragments (total 2-3 mm³) on each flank of the mouse. The wound was closed using Permacol cyanoacrylate glue.

At the end of experiment the mice were sacrificed by CO₂ inhalation. The synovial grafts were explanted. One of them was snap-frozen in OCT compound (TissueTek, Sacura Finetek Europe) for further immunohistochemical analysis.

### Example 2 - Serum collection

Blood samples were collected using capillaries (Microvette CB300, Sarstedt, Germany) before and weekly after implantation. To ensure larger volume of serum, at the end of the experiment the mice were sacrificed by CO₂ inhalation and blood samples were collected by heart punction using a 25 G needle on a 1 ml syringe. Serum was obtained by centrifugation in an Eppendorf MiniSpin centrifuge for 30 min at 13000 rpm and stored at -20 °C.

### Example 3 - Immunohistochemistry

5 µM cryosections on SuperFrost (Menzel GmbH, Braunschweig, Germany) slides were prepared using LEICA CM1900 cryostate and stored at -80 °C. Thawed sections were fixed in acetone for 10 min, dried at room temperature and washed 3 x 5 min in PBS (phosphate buffered saline). All steps were performed at room temperature. Endogenous peroxidase activity was blocked by incubation with 0.3% hydrogen peroxide / 0.1% sodium azide for 20 min. Slides were washed 3 x 5 min in PBS. Thereafter, primary antibody diluted in 1% BSA (bovine serum albumin)/ PBS was incubated for 60 min. After 3 x 2 min wash in PBS, HRP (horseradish peroxidase)-conjugate (goat anti-mouse Ig-HRP; DAKO P0447, DAKO, Glostrup, Denmark) diluted 1:50 in 1% BSA / 10% NHS / PBS was added over 30 min. Peroxidase signal was enhanced using TSA™ Biotin system (Perkin Elmer Life Sciences, NEL700). Shortly, slides were washed 3 x 2 min in PBS and incubated with biotinyl tyramide diluted 1:400 in amplification buffer for 30 min. After 3 x 2 min wash in PBS, streptavidin-HRP diluted 1:100 in 1% BSA / PBS was added for 30 min. Slides were washed 3 x 2 min in PBS and preincubated with AEC (3-amino-9-ethylcarbazole) buffer (0.03M acetic acid / 0.07M sodium acetate) for 2 min. Peroxidase activity was detected using AEC as a substrate. Slides were incubated with the AEC solution (0.47 mg/ml AEC / 0.22% hydrogen peroxide in AEC buffer) for 20 min. Colour reaction was stopped with distilled water. Finally, slides were counterstained with hematoxyline (Merck), washed with running water and covered with Kaiser's glycerin and cover slips.

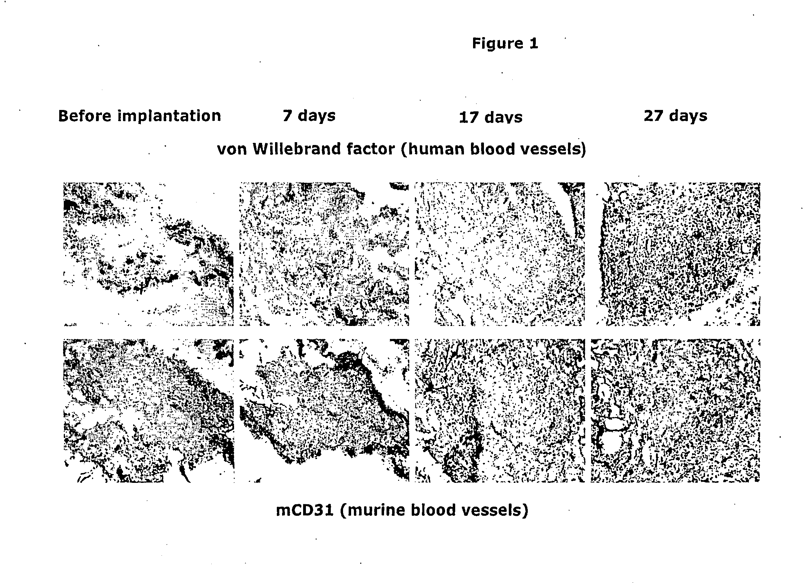

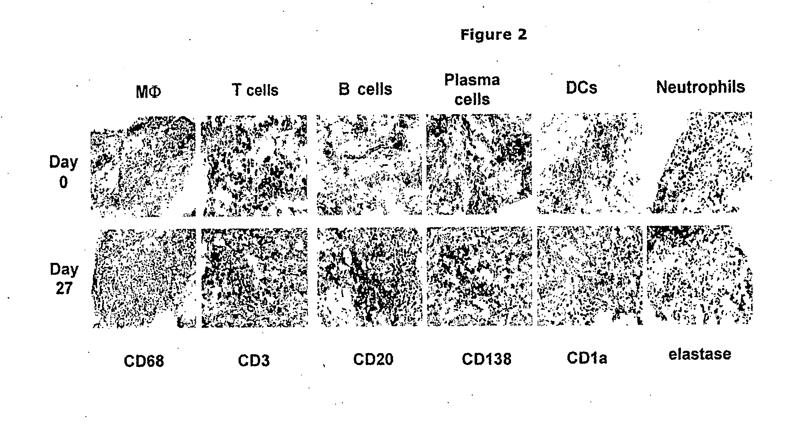

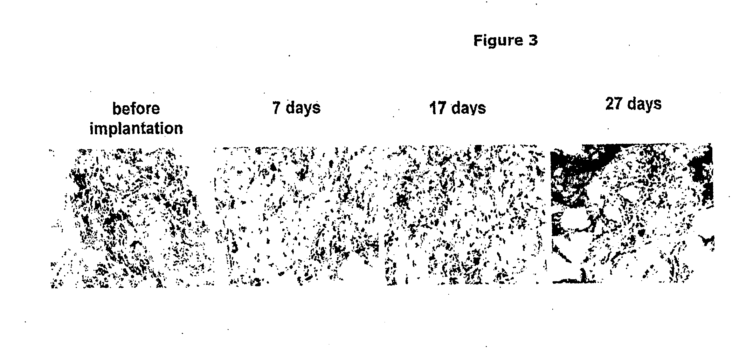

As it appears from Figures 1 to 3 the inflamed synovial tissue of the RA patient was successfully engrafted. As it can be seen from Figure 1 the graft becomes vascularized by murine vessels. Figure 2 shows that human immune cells, including T cells, B cells, plasma cells, macrophages, dendritic cells, but not neutrophils, persist in the graft for a prolonged time, i.e. at least up to 3 weeks after implantation. Figure 3 shows the persistence of cellular infiltrate in the xenograft for a prolonged time, i.e. at least up to 3 weeks after implantation.

### Example 4 - Anti-CCP antibody assay

The presence of human anti-CCP antibodies in sera was measured using anti-CCP-ELISA (CCP1 test, cf. Schellekens GA, et al. (2000) "The diagnostic properties of rheumatoid arthritis antibodies recognizing a cyclic citrullinated peptide" Arthritis Rheum 43: 155-163). Plates coated with cyclic peptide containing arginine or citrullinated cyclic peptide (CCP; obtained from Dr W. van Venrooij, Dept. Biochemistry, NCMLS Nijmegen, the Netherlands) were incubated with 100 µl/well of mouse sera diluted 1:4 in PBS / 1% BSA / 0.05% Tween-20 for 1 hour at 37 °C in a humid incubation chamber. Anti-CCP-positive human serum was used as a positive control. After wash (6 x) with PBS / 0.05% Tween-20, 100 µl of rabbit anti-human HRP-conjugated antibody (P0214, DAKO, Glostrup, Denmark; 1:10000 in PBS / 1% BSA / 0.05% Tween-20) was added to each well. The plates were incubated for 1 hour at 37 °C in a humid incubation chamber and washed 6 times with PBS / 0.05% Tween-20. Bound antibodies were visualized using 100 µl/well of 3,3',5,5'-tetramethylbenzidine (TMB) / ureumperoxide at room temperature. The staining reaction was stopped with 100 µl/well of 2M H₂SO₄. OD450 was read using an ELISA reader (EL 312e, Bio-Tek Instruments).

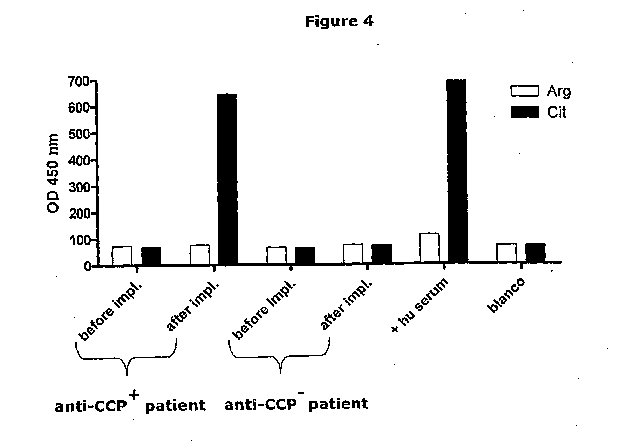

The CCP1 test allows detection of human anti-CCP antibodies in the SCID-mice implanted with human synovial tissue. As it can be seen from Figure 4 sera of mice implanted with synovium of an anti-CCP+ RA patient become anti-CCP+ within the first two weeks after implantation. Sera of mice implanted with synovium of anti-CCP- patient remain anti-CCP-.

### Example 5 - Effect of antibody treatment on tissue graft B cells and anti-CCP antibodies in an RA SCID mouse model

Implantation of synovial tissue: SCID-mice, strain C.B.-17/IcrCrl-SCID-bg, male / female, 4-12 weeks, are kept in IVC cages under standard conditions of temperature and light, and fed laboratory chow and water ad libitum. Prior to implantation the mice (for example three mice in each experimental group, day 0) are anesthetized by intraperitoneal injection of ketamine (NIMATEK, EuroVet) and xylazine (Rompun, Bayer) at a ratio of 1:1 v/v. A small incision of the skin is made using surgical scissors. Inflamed synovial tissue of a patient with RA undergoing joint replacement surgery is implanted subcutaneously as two clusters of up to six small fragments (total 2-3 mm³) on each flank of the mouse. The wound is closed using Permacol cyanoacrylate glue.

On day one of the experiment, remaining synovial tissue is analyzed in order to control for B cells in the inflamed synovial transplants.

On day 8 of the experiment, purified monoclonal antibodies such as an monoclonal antibody against CD38, CD20, IL-15, IL-1R, IL-6Ralpha or gp130 is injected i.v. in a volume of 200 µl at a dose of 10 to 30 mg/kg.

In one experimental set-up, the mice are sacrificed on day 14 by CO₂ inhalation and the synovial grafts are explanted. The grafts are snap-frozen in OCT compound (TissueTek, Sacura Finetek Europe) for immunohistochemical analysis.

In a second experimental set up, the antibody titers are followed over longer periods of time, for example of up to 49 days or longer, during which time serum is collected weekly and the antibody titers determined.

Serum collection: Blood samples are collected using capillaries (Microvette CB300, Sarstedt, Germany) before (day 0) and weekly (day 7 and day 14) after implantation for the first experimental set up or at day 0, 7, 14, 21, 28, 35, 42, and 49 for the second experimental set up. To ensure larger volume of serum, at the end of the experiment, the mice are sacrificed by CO₂ inhalation and blood samples are collected by heart puncture using a 25 G needle on a 1 ml syringe. Serum is obtained by centrifugation in the Eppendorf MiniSpin centrifuge for 30 min at 13,000 rpm and stored at -20 °C until the assay is performed.

Immunohistochemistry: 5 µM cryosections on SuperFrost (Menzel GmbH, Braunschweig) slides are prepared using LEICA CM1900 cryostate and stored at -80 °C. Thawed sections are fixed in acetone for 10 min, dried at room temperature and washed 3 x 5 min in PBS. All steps are performed at room temperature. Endogenous peroxidase activity is blocked by incubation with PBS supplemented with 0.3% hydrogen peroxide and 0.1% sodium azide for 20 min. Slides are washed 3 x 5 min in PBS. Next, primary antibody diluted in PBS supplemented with 1% BSA is incubated for 60 min. After 3 x 2 min wash in PBS, HRP-conjugate (goat anti-mouse Ig-HRP; DAKO P0447) diluted 1:50 in PBS (supplemented with 1% BSA and 10% NHS) for 30 min is added for 30 min. Peroxidase signal is enhanced using TSA™ Biotin system (Perkin Elmer Life Sciences, NEL700). Shortly, slides are washed 3 x 2 min in PBS and incubated with biotinyl tyramide diluted 1:400 in amplification buffer for 30 min. After 3 x 2 min wash in PBS, streptavidin-HRP diluted 1:100 in PBS (supplemented with 1% BSA) is added for 30 min. Slides are washed 3 x 2 min in PBS and preincubated with AEC buffer (0.03M acetic acid / 0.07M sodium acetate) for 2 min. Peroxidase activity is detected using AEC as a substrate: slides are incubated with the AEC solution (0.47 mg/ml AEC / 0.22% hydrogen peroxide in AEC buffer) for 20 min. Color reaction is stopped with distilled water. Finally, slides are counterstained with hematoxyline (Merck), washed with running water and covered with Kaiser's glycerin and cover slips.

Scoring of staining intensity: Scoring of stained synovial tissue xenografts is performed in a blinded fashion by two persons. A score on a scale of 0-8 is used, and this scoring is performed relative to a positive control on a section from the same animal.

Detection of anti-CCP antibody production by human antibody producing cells from the implanted tissue graft: The production of anti-CCP antibodies can be detected by a specific ELISA.

Statistical analysis: Scoring of staining intensity is analyzed by Kruskal-Wallis one-way ANOVA followed by Dunn's multiple comparison test using Graph Pad Prism version 4.01 (Graph Pad software, Inc., San Diego, CA, USA).

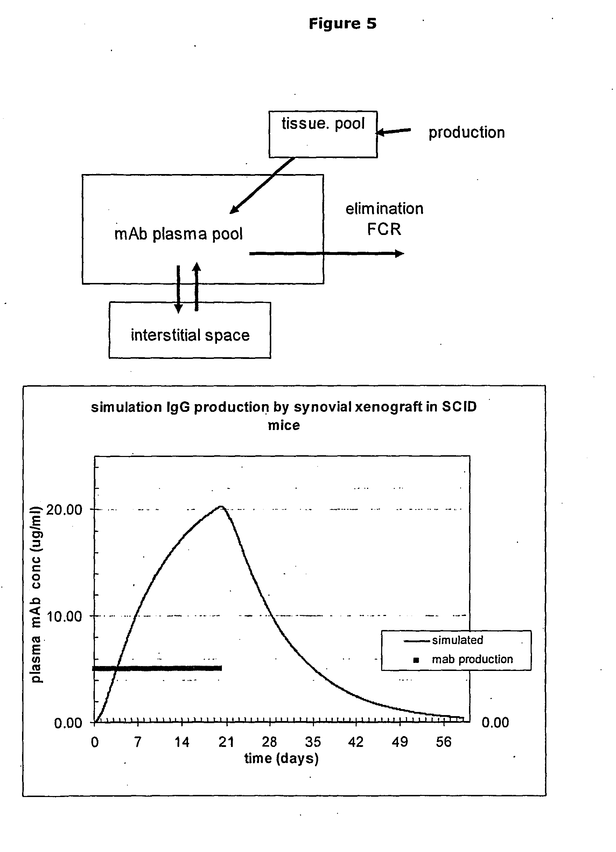

Modeling of antibody pharmacokinetics following transplantation of the SCID mice with human synovial tissue harboring anti-CCP antibody-producing cells: The plasma concentration of IgG including human anti-CCP antibodies secreted by antibody-producing cells from synovial tissue transplants in SCID mice is simulated in Figure 5. The antibody production takes place in the synovial tissue grafts or by cells migrated from the graft into the mouse tissues or circulation. It is assumed that locally produced antibody equilibrates with the plasma compartment with a half-life (t_{½}) of 12 hours. From there, half of the plasma pool is redistributed into the interstitial space with a t_{½} of 3 hours. Elimination from the mouse plasma compartment occurs with a t_{½} of 10 days (which is a normal value for SCID mice). In this simulation the IgG production rate is set at 200 µg/kg/day which corresponds to a production of about 200 ng IgG per hour per synovial transplant. Anti-CCP antibodies form part of the IgG, and will follow the same kinetics. Higher or lower antibody production may be expected if tissue grafts containing smaller or greater numbers of antibody-producing cells are transplanted. Under the assumption that antibody production takes place during a period of 21 days, IgG (including anti-CCP) concentrations of 20 µg/ml in the SCID mouse serum are readily achieved (see simulation in Figure 5). Treatment with the passively transferred antibodies, such as anti-CD38, anti-CD20 or anti-IL-15 antibodies performed during the first 14 days, such as on day 8, would then inhibit antibody production from that day forward and reductions in anti-CCP antibody production are readily detected in ELISA as discussed above.

The present example is a minimal scenario and it is expected that graft survival and anti-CCP antibody production will be observed for longer periods of time. In such case there is an extended period of antibody build-up, and higher antibody plasma concentrations are achieved. The window for immunotherapeutic approaches with antibodies will thus extend proportionally.

## Claims

1. Use of a non-human mammalian disease model, wherein the non-human mammal has been implanted with human synovial tissue or other human inflamed tissue containing anti-CCP antibody producing cells, wherein anti-CCP antibodies are present in the serum of the non-human mammal, for testing the efficacy of an anti-CD20 antibody for the prevention or treatment of RA (rheumatoid arthritis) or for identifying an anti-CD20 antibody useful for the prevention or treatment of RA, wherein the efficacy of the anti-CD20 antibody is measured or the anti-CD20 antibody is identified by its ability to reduce the concentration or activity of anti-CCP antibodies in the serum of the non-human mammalian model.

2. Use according to claim 1, wherein the antibody is an anti-CD20 antibody selected from the group consisting of the antibodies 2F2, 7D8 11B8, rituximab, tositumomab, 2H7.V16, IMMU-106, or TRU-015.

3. Use according to any one of the preceding claims, wherein the non-human mammal is a mouse.

4. Use according to claim 3, wherein the non-human mammal is a SCID mouse or a nude mouse.

5. Use according to any one of the preceding claims, wherein the non-human mammal has been implanted with human synovial tissue or other human inflamed tissue containing anti-CCP antibody producing cells derived from a patient suffering from RA.

6. Use according to any one of the preceding claims, wherein an agent for stimulating anti-CCP antibody production has been added to the human synovial tissue or other human inflamed tissue containing anti-CCP antibody producing cells prior to implantation thereof.

7. Use according to claim 6, wherein the agent for stimulating anti-CCP antibody production is selected from the group consisting of LPS (lipopolysaccharide) and T cell-derived cytokines, such as IL-4, IFN-Y (interferon- gamma) or IL-10.

8. Use according to any one of the preceding claims, wherein the non-human mammal has been administered with an agent for stimulating anti-CCP antibody production prior to, during or after implantation of the human synovial tissue or other human inflamed tissue containing anti-CCP antibody producing cells.

9. Use according to claim 8, wherein the agent for stimulating anti-CCP antibody production is selected from the group consisting of LPS (lipopolysaccharide) and T cell-derived cytokines, such as IL-4, IFN-Y (interferon- gamma) or IL-10.

10. Use according to any one of the preceding claims, wherein the non-human mammal has been implanted with human synovial tissue or other human inflamed tissue containing anti-CCP antibody producing cells, wherein arthritis is induced by intra-articular injection of an inflammation inducing agent.

11. Use according to claim 10, wherein the inflammation inducing agent is selected from the group consisting of LPS, streptococcal wall-debris, immune complexes, cytokine-producing recombinant viruses, including recombinant adenovirus, recombinant adeno-associated virus, and recombinant retroviruses.

12. Use according to any one of the preceding claims, wherein the non-human mammal has been implanted with human synovial tissue or other human inflamed tissue containing anti-CCP antibody producing cells, wherein citrullinated proteins are generated in the articular joint by intra-articular injection of recombinant viruses encoding citrunillating enzymes of human, vertebrate or non-vertebrate origin, including peptidyl arginine deiminase 1-6, or by injection of other vectors encoding such enzymes, or by injection of protein representing such enzymes.

## Patentansprüche

1. Verwendung eines nicht-humanen Säugetier-Modells für eine Krankheit, bei der dem nicht-humanen Säugetier ein humanes Synovialgewebe oder ein anderes humanes entzündetes Gewebe, welches Anti-CCP-Antikörper erzeugende Zellen enthält, implantiert wurde, wobei die Anti-CCP-Antikörper in dem Serum des nicht-humanen Säugetieres vorhanden sind, um die Wirksamkeit eines Anti-CD20-Antikörpers für die Prävention oder Behandlung von RA (rheumatoide Arthritis) zu testen oder um einen Anti-CD20-Antikörper, der für die Prävention oder Behandlung von RA verwendbar ist, zu identifizieren, und wobei die Wirksamkeit des Anti-CD20-Antikörpers gemessen oder der Anti-CD20-Antikörper identifiziert wird anhand seiner Fähigkeit, die Konzentration oder die Aktivität der Anti-CCP-Antikörper in dem Serum des nicht-humanen Säugetier-Modells zu verringern.

2. Verwendung nach Anspruch 1, bei der der Antikörper ein Anti-CD20-Antikörper ist, der aus einer Gruppe bestehend aus den Antikörpern 2F2, 7D8, 11 B8, Rituximab, Tositumomab, 2H7.V16, IMMU-106, oder TRU-015 ausgewählt ist.

3. Verwendung nach einem der vorhergehenden Ansprüche, bei der das nicht-humane Säugetier eine Maus ist.

4. Verwendung nach Anspruch 3, wobei das nicht-humane Säugetier eine SCID-Maus oder eine Nacktmaus ist.

5. Verwendung nach einem der vorhergehenden Ansprüche, bei der dem nicht-humanen Säugetier ein humanes Synovialgewebe oder ein anderes humanes entzündetes Gewebe implantiert wurde, das Anti-CCP-Antikörper erzeugende Zellen enthält, die von einem Patienten stammen, der unter RA leidet.

6. Verwendung nach einem der vorhergehenden Ansprüche, bei der ein Mittel zur Stimulierung der Anti-CCP-Antikörper-Produktion dem humanen Synovialgewebe oder dem anderen humanen entzündeten Gewebe, die die Anti-CCP-Antikörper erzeugenden Zellen enthalten, beigegeben wurde, und zwar vor deren Implantierung.

7. Verwendung nach Anspruch 6, bei der das Mittel zur Stimulierung der Anti-CCP-Antikörper-Produktion ausgewählt ist aus der Gruppe bestehend aus LPS (Lipopolysaccharide) und von T-Zellen stammende Zytokine, wie etwa IL-4, IFN-Y (Interferongamma) oder IL-10.

8. Verwendung nach einem der vorhergehenden Ansprüche, bei der dem nicht-humanen Säugetier ein Mittel zur Stimulierung der Anti-CCP-Antikörper-Produktion vor, während oder nach der Implantation des humanen Synovialgewebes oder des anderen humanen entzündeten Gewebes, welches Anti-CCP-Antikörper erzeugende Zellen enthält, verabreicht wurde.

9. Verfahren nach Anspruch 8, bei der das Mittel zur Stimulierung der Anti-CCP-Antikörper-Produktion ausgewählt ist aus einer Gruppe bestehend aus LPS (Lipopolysaccharide) und von T-Zellen stammende Zytokine, wie etwa IL-4, IFN-Y (Interferon-gamma) oder IL-10.

10. Verwendung nach einem der vorhergehenden Ansprüche, bei der dem nicht-humanen Säugetier ein humanes Synovialgewebe oder ein anderes humanes entzündetes Gewebe, welches Anti-CCP-Antikörper erzeugende Zellen enthält, implantiert wurde, wobei eine Arthritis durch intraartikuläre Injektion eines entzündungsinduzierenden Mittels induziert wird.

11. Verwendung nach Anspruch 10, bei der das entzündungsinduzierende Mittel ausgewählt ist aus der Gruppe bestehend aus LPS, Streptokokken-Wandablagerungen, Immunkomplexe, Zytokin erzeugende rekombinante Viren, umfassend rekombinante Adenoviren, rekombinante Adeno-assoziierte Viren, und rekombinante Retroviren.

12. Verwendung nach einem der vorhergehenden Ansprüche, bei der dem nicht-humanen Säugetier ein humanes Synovialgewebe oder ein anderes humanes entzündetes Gewebe, das Anti-CCP-Antikörper erzeugende Zellen enthält, implantiert wurde, wobei citrullinierte Proteine in dem Gelenk durch intraartikuläre Injektion von rekombinanten Viren, die die citrullinierenden Enzyme des Menschen entschlüsseln, die von einem Wirbeltier oder einem Nicht-Wirbeltier stammen, und die Peptidylarginin Deiminase 1-6 umfassen, oder durch Injektion anderer Vektoren, die solche Enzyme entschlüsseln, oder durch Injektion von Proteinen, die solche Enzyme bilden, erzeugt werden.

## Revendications

1. Utilisation d'un modèle de maladie de mammifère non humain, où le mammifère non humain a été implanté avec du tissu synovial humain ou un autre tissu enflammé humain contenant des cellules productrices d'anticorps anti-CCP, les anticorps anti-CCP étant présents dans le sérum du mammifère non humain, pour l'analyse de l'efficacité d'un anticorps anti-CD20 pour la prévention ou le traitement de la polyarthrite rhumatoïde (PR) ou pour l'identification d'un anticorps anti-CD20 utile pour la prévention ou le traitement de la PR, où l'efficacité de l'anticorps anti-CD20 est mesurée ou l'anticorps anti-CD20 est identifié par sa capacité à réduire la concentration ou l'activité des anticorps anti-CCP dans le sérum du modèle de mammifère non humain.

2. Utilisation selon la revendication 1, où l'anticorps est un anticorps anti-CD20 choisi dans le groupe constitué des anticorps 2F2, 7D8, 11B8, rituximab, tositumomab, 2H7.V16, IMMU-106 ou TRU-015.

3. Utilisation selon l'une quelconque des revendications précédentes, où le mammifère non humain est une souris.

4. Utilisation selon la revendication 3, où le mammifère non humain est une souris SCID ou une souris nude.

5. Utilisation selon l'une quelconque des revendications précédentes, où le mammifère non humain a été implanté avec du tissu synovial humain ou un autre tissu enflammé humain contenant des cellules productrices d'anticorps anti-CCP dérivé d'un patient souffrant de PR.

6. Utilisation selon l'une quelconque des revendications précédentes, où un agent destiné à la stimulation de la production d'anticorps anti-CCP a été ajouté au tissu synovial humain ou à l'autre tissu enflammé humain contenant des cellules productrices d'anticorps anti-CCP avant son implantation.

7. Utilisation selon la revendication 6, où l'agent destiné à la stimulation de la production d'anticorps anti-CCP est choisi dans le groupe constitué de LPS (lipopolysaccharide) et de cytokines dérivées des lymphocytes T, comme l'IL-4, l'IFN-γ (interféron gamma) ou l'IL-10.

8. Utilisation selon l'une quelconque des revendications précédentes, où le mammifère non humain a reçu l'administration d'un agent destiné à la stimulation de la production d'anticorps anti-CCP avant, durant ou après l'implantation du tissu synovial humain ou d'un autre tissu enflammé humain contenant des cellules productrices d'anticorps anti-CCP.

9. Utilisation selon la revendication 8, où l'agent destiné à la stimulation de la production d'anticorps anti-CCP est choisi dans le groupe constitué de LPS (lipopolysaccharide) et de cytokines dérivées des lymphocytes T, comme l'IL-4, l'IFN-γ (interféron gamma) ou l'IL-10.

10. Utilisation selon l'une quelconque des revendications précédentes, où le mammifère non humain a été implanté avec du tissu synovial humain ou un autre tissu enflammé humain contenant des cellules productrices d'anticorps anti-CCP, où une arthrite est induite par injection intra-articulaire d'un agent inducteur d'inflammation.

11. Utilisation selon la revendication 10, où l'agent inducteur d'inflammation est choisi dans le groupe constitué de LPS, de débris de la paroi streptococcique, de complexes immuns, de virus recombinants producteurs de cytokines, y compris des adénovirus recombinants, des virus adéno-associés recombinants, et des rétrovirus recombinants.

12. Utilisation selon l'une quelconque des revendications précédentes, où le mammifère non humain a été implanté avec du tissu synovial humain ou un autre tissu enflammé humain contenant des cellules productrices d'anticorps anti-CCP, où des protéines citrullinées sont générées dans l'articulation par injection intra-articulaire de virus recombinants codant pour des enzymes citrullinantes d'origine humaine, de vertébré ou de non vertébré, y compris les peptidyl-arginine désiminases 1 à 6, ou par injection d'autres vecteurs codant pour de telles enzymes, ou par injection de protéines représentant de telles enzymes.
